# EUROPÄISCHE PATENTANMELDUNG

(11) **EP 0 238 792 A1**
(43) Veröffentlichungstag der Anmeldung: **30.09.1987**
(21) Anmeldenummer: 87100648.2
(22) Anmeldetag: 19.01.1987
(51) Int. Cl.: C07C 79/35, C07C 76/02, C07C 149/32

(54) **Verfahren zur Herstellung von Halogenalkoxy-diphenylethern**

(30) Priorität: 30.01.1986 DE 3602680
(71) Anmelder: BAYER AG, 51368 Leverkusen (DE)
(72) Erfinder: Gallenkamp, Bernd, Dr., D-5600 Wuppertal 1 (DE); Marhold, Albrecht, Dr., D-5090 Leverkusen 1 (DE); Sirrenberg, Wilhelm, Dr., D-4322 Sprockhövel (DE)

(57) **Zusammenfassung**

Die Erfindung betrifft ein neues mehrstufiges Verfahren zur Herstellung von Halogenalkoxy-diphenylethern der Formel (1) 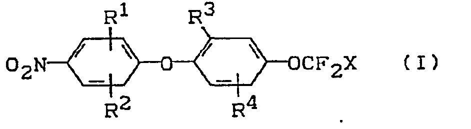 in welcher
R¹ für Wasserstoff, Halogen, Alkyl, Alkoxy oder Alkylthio steht,
R² für Wasserstoff, Halogen, Alkyl, Alkoxy oder Alkylthio steht,
R³ für Wasserstoff, Chlor oder Brom steht,
R⁴ für Wasserstoff oder Alkyl steht und
X für Wasserstoff, Fluor, Chlor oder die Reste -CH₂Cl, -CHF₂, -CHCl₂ und -CHFCI steht.

## Beschreibung

Die Erfindung betrifft ein neues Verfahren zur Herstellung von Halogenalkoxy-diphenylethern, welche als Zwischenprodukte für die Herstellung von Pestiziden wie insbesondere Insektiziden und Herbiziden verwendet werden.

Es ist bereits bekannt, daß man Halogenalkoxy-diphenylether herstellen kann, wenn man Halogenbenzole mit Halogenalkoxyphenolen umsetzt (vergl. z. B. US-PS 4 192 669). Diese Herstellungsmethode ist jedoch insbesondere wegen unbefriedigender Herstellbarkeit der Halogenalkoxyphenole nur begrenzt anwendbar. Es besteht daher Bedarf an neuen Verfahren zur Herstellung dieser wichtigen Zwischenprodukte.

Es wurden nun gefunden, daß man die Halogenalkoxy-diphenylether der Formel (1) 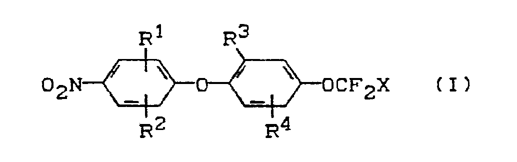 in welcher
R¹ für Wasserstoff, Halogen, Alkyl, Alkoxy oder Alkylthio steht,
R² für Wasserstoff, Halogen, Alkyl, Alkoxy oder Alkylthio steht,
R³ für Wasserstoff, Chlor oder Brom steht,
R⁴ für Wasserstoff oder Alkyl steht und
X für Wasserstoff, Fluor, Chlor oder die Reste -CH₂C1, -GHF₂, -CHCl₂ und -CHFC1 steht,

erhält, wenn man
(a) Halogenbenzole der allgemeinen Formel (II) 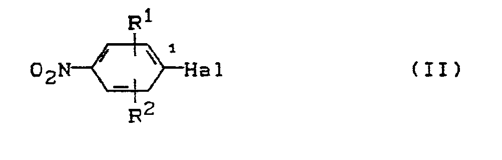 in welcher
   R¹ und R² die oben angegebenen Bedeutungen haben und
   Hal für Halogen steht,
   mit Hydrochinon-Derivaten der allgemeinen Formel (III) 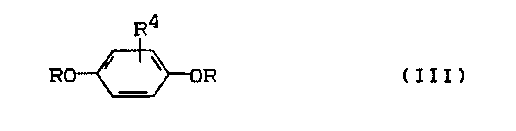 in welcher
   R⁴ die oben angegebenen Bedeutungen hat und
   R für Wasserstoff oder ein Äquivalent eines Alkali- oder Erdalkalimetallkations steht,
   gegebenenfalls in Gegenwart von Säureakzeptoren und in Gegenwart von inerten Verdünnungsmitteln bei Temperaturen,zwischen 70 °C und 140 °C umsetzt, gegebenenfalls mit Mineralsäuren versetzt zu den Hydrochinonmonophenylethern der allgemeinen Formel (IV) 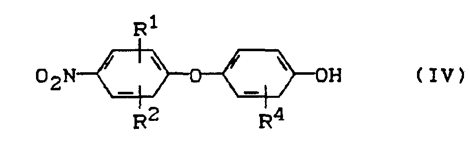 in welcher
   R¹, R² und R⁴ die oben angegebenen Bedeutungen haben,
   und anschließend
(b) die Verbindungen der Formel (IV), nach ihrer Isolierung, mit halogenierten gesättigten oder ungesättigten Kohlenwasserstoffen, gegebenenfalls in Gegenwart von Fluorwasserstoff, gegebenenfalls in Gegenwart von Basen, gegebenenfalls in Gegenwart von Katalysatoren und gegebenenfalls in Gegenwart von Verdünnungsmitteln bei Temperaturen zwischen 0 °C und 160 °C zu den Verbindungen der Formel (Ia) 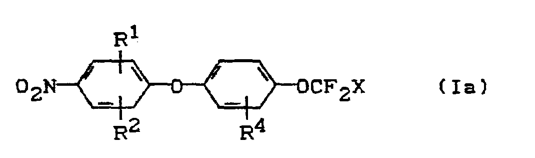 in welcher
   X, R^{l}, R² und R⁴ die oben angegebenen Bedeutungen haben,
   umsetzt und gegebenenfalls anschließend
(c) die Verbindungen der Formel (Ia), nach ihrer Isolierung, in Gegenwart von Katalysatoren und in Gegenwart von polaren Verdünnungsmitteln bei Temperaturen zwischen 0 °C und 100 °C halogeniert und die Verbindungen der Formel (Ib) 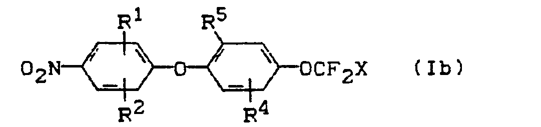 in welcher
   X, R¹, R² und R⁴ die oben angegebenen Bedeutungen haben und
   R⁵ für Chlor oder Brom steht,

   isoliert.

Die Verbindungen der Formel (I), in welchen R für Wasserstoff steht, werden als Verbindungen der Formel (Ia) bezeichnet und die Verbindungen der Formel (I), in welchen R für Chlor oder Brom steht, werden als Verbindungen der Formel (Ib) bezeichnet.

Gemäß diesem Verfahren ist es möglich, die Verbindungen der Formel (I) auf einfache Weise in guter Reinheit herzustellen. Das Verfahren ist im Hinblick auf die Art der gewünschten Substituenten sehr breit anwendbar. Weiterhin sind die als Zwischenprodukte einzusetzenden Verbindungen stabil und können gut gelagert und gehandhabt werden.

Alkyl _{R}¹, R² und R⁴ stehen für geradkettiges oder verzweigtes Alkyl mit 1 bis 12, vorzugsweise 1 bis 6, insbesondere 1 bis 4 Kohlenstoffatomen. Beispielhaft seien Methyl, Ethyl, n-Propyl, i-Propyl, n-Butyl, i-Butyl, sec.-Butyl und tert.-Butyl und besonders bevorzugt Methyl genannt.

Alkoxy R¹ und R² stehen für geradkettiges oder verzweigtes Alkoxy mit 1 bis 12, vorzugsweise 1 bis 6, insbesondere 1 bis 4 Kohlenstoffatomen. Beispielhaft seien Methoxy, Ethoxy, n-Propoxy, i-Propoxy, n-Butoxy, i-Butoxy, sec.-Butoxy und tert.-Butoxy genannt.

Alkylthio R^{l} und R² stehen für geradkettiges oder verzweigtes Alkylthio mit 1 bis 12, vorzugsweise 1 bis 6, insbesondere 1 bis 4 Kohlenstoffatomen. Beispielhaft seien Methylthio, Ethylthio, n-Propylthio, i-Propylthio, n-Butylthio, i-Butylthio, sec.-Butylthio und tert.-Butylthio genannt.

Halogen bedeutet (wo nicht anders erläutert) Fluor, Chlor, Brom und Iod, vorzugsweise Fluor, Chlor und Brom.

Vorzugsweise werden mit Hilfe des erfindungsgemäßen Verfahrens die Verbindungen der Formel (I) hergestellt, worin
R¹ für Wasserstoff, Halogen wie Fluor, Chlor oder Brom und für Alkyl, Alkoxy oder Alkylthio mit jeweils 1 bis 6 Kohlenstoffatomen steht,
R² für Wasserstoff, Halogen wie Fluor, Chlor oder Brom und für Alkyl, Alkoxy oder Alkylthio mit jeweils 1 bis 6 Kohlenstoffatomen steht,
R³ für Wasserstoff, Chlor oder Brom steht,
R⁴ für Wasserstoff oder Alkyl mit 1 bis 6 Kohlenstoffatomen steht und
X für Wasserstoff, Fluor, Chlor oder die Reste -CH₂Cl, -CHF₂, -CHCl₂ und -CHFC1 steht.

Besonders bevorzugt werden die Verbindungen der Formel (I) hergestellt, in welcher
R¹ für Wasserstoff, Fluor, Chlor, Brom oder für Alkyl, Alkoxy oder Alkylthio mit jeweils 1 bis 4 Kohlenstoffatomen steht,
R² für Wasserstoff, Fluor, Chlor, Brom oder für Alkyl, Alkoxy oder Alkylthio mit jeweils 1 bis 4 Kohlenstoffatomen steht,
R³ für Wasserstoff, Chlor oder Brom steht,
R⁴ für Wasserstoff, oder Alkyl mit 1 bis 4 Kohlenstoffatomen steht und
X für Wasserstoff, Fluor, Chlor oder die Reste -CH₂C1, -CHF₂, -CHC1₂ und -CHFC1 steht.

Ganz besonders bevorzugt werden die Verbindungen der Formel (I) hergestellt, in welcher
R¹ für Wasserstoff, Fluor, Chlor, Methyl, Methoxy und Methylthio (vorzugsweise Methyl) steht,
R² für Wasserstoff, Fluor, Chlor, Methyl, Methoxy und Methylthio (vorzugsweise Wasserstoff oder Methyl) steht,
R³ für Wasserstoff oder Chlor steht,
R⁴ für Wasserstoff, Methyl oder Ethyl (vorzugsweise Wasserstoff) steht und
X für Wasserstoff, Fluor, Chlor oder die Reste -CH2C1, -CHF_{Z}, -CHCl₂ und -CHFC1 (vorzugsweise Wasserstoff, Fluor, Chlor oder CHFC1) steht.

Besonders bevorzugt stehen die Reste R¹ und R² in den 2-und 6-Stellungen des 4-Nitrophenylrestes.

Die Erläuterung der bevorzugten Restedefinitionen gilt für alle aufgeführten allgemeinen Formeln sinngemäß.

Die Herstellung der Verbindungen der Formel (IV) bzw. ihre Herstellung gemäß Verfahrensschritt (a) ist Teil der vorliegenden Erfindung.

Es wurde demgemäß gefunden, daß man Hydrochinonmonophenylether der allgemeinen Formel (IV) 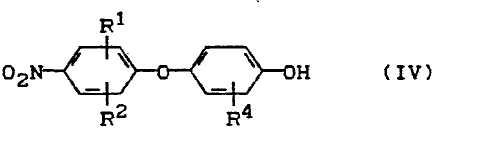 in welcher
R¹, R² und R⁴ die oben angegebenen Bedeutungen haben,
erhält, wenn man Halogenbenzole der allgemeinen Formel (II) 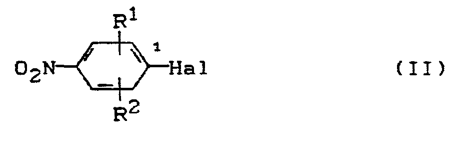
R¹, R² und Hal die oben angegebenen Bedeutungen haben,
mit Hydrochinon-Derivaten der allgemeinen Formel (111) 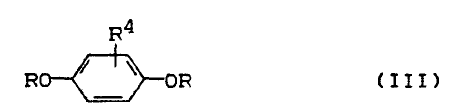 in welcher
R und R⁴ die oben angegebenen Bedeutungen haben,

gegebenenfalls in Gegenwart von Säureakzeptoren und in Gegenwart von inerten Verdünnungsmitteln bei Temperaturen zwischen 70 °C und 140 °C umsetzt, anschließend gegebenenfalls mit Mineralsäuren versetzt und die Verbindungen der Formel IV isoliert.

Überraschenderweise ist es möglich mit Hilfe des erfindungsgemäßen Verfahrens unter relativ milden Bedingungen die Hydrochinonmonophenylether der Formel (IV) in guter Ausbeute und sehr hoher Reinheit zu erhalten. Weitere Vorteile des Verfahrens bestehen darin daß eine destillative Entfernung von gebildetem Wasser nicht notwendig ist (vergl. z. B. DE-OS 24 33 066 und 30 18 004).

Verwendet man für das erfindungsgemäße Verfahren bzw. den erfindungsgemäßen Verfahrensschritt (a) 2-Methyl-4-nitrobenzol und Hydrochinon als Ausgangsstoffe und Kaliumhydroxid als Säureakzeptor oder 2-Methyl-4-nitrobenzol und das Dikaliumsalz des Hydrochinons als Ausgangsstoffe und Salzsäure als Mineralsäure, so können die Reaktionen durch die folgenden Formelschemata skizziert werden:

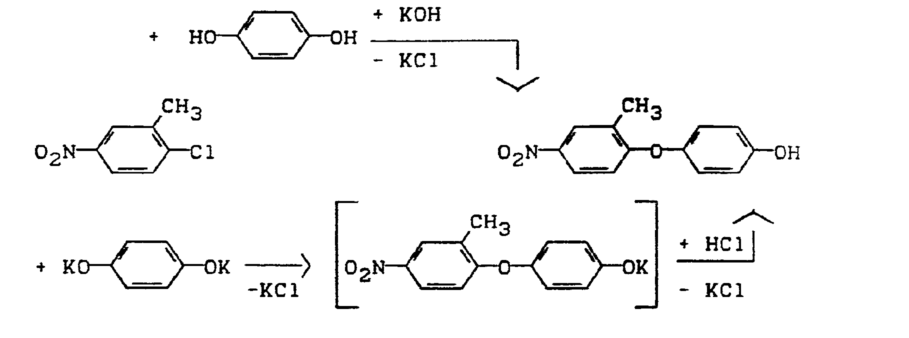

Die beim Verfahrensschritt (a) einzusetzenden Halogenbenzole sind durch die Formel (II) allgemein definiert. In dieser Formel stehen R und R² vorzugsweise für diejenigen Reste, die bereits im Zusammenhang mit der Beschreibung der Verbindungen der Formel (I) genannt wurden. Ha1 steht in dieser Formel (II) für Halogen, vorzugsweise für Fluor, Chlor oder Brom.

Die Verbindungen der Formel (II) sind allgemein bekannte Verbindungen der organischen Chemie.

Als Beispiele für die Halogenbenzole der Formel (II) seien genannt:
4-Nitro-chlorbenzol, 4-Nitro-fluorbenzol, 4-Nitro-brombenzol, 2-Methyl-, 2-Chlor-, 2-Fluor-, 2-Brom-, 2-Methoxy-,
2-Methylthio-, 2-Ethyl-, 2-Ethoxy-, 2-Ethylthio-, 2,6-Dimethyl-, 2-Chlor-6-methyl-, 2-Fluor-6-methyl-, 2-Methyl-6-methoxy, 2-Methyl-6-methylthio-, 3-Methyl-, 3-Ethyl-, 3-Ethoxy-, 3-Ethylthio-, 3-Chlor-, 3-Fluor-, 3-Brom-, 3-Methoxy-, 3-Methylthio-, 2,3-Dimethyl-, 3,5-Dimethyl-, 2,5-Dimethyl-, 2,6-Dichlor-, 2,6-Difluor-, 3,5-Dichlor-, 2,6-

Dimethoxy-4-nitro-chlorbenzol bzw. -4-nitro-brombenzo1 und -4-nitro-fluorbenzol.

Die beim erfindungsgemäßen Verfahrensschritt (a) außerdem als Ausgangsstoffe zu verwendenden Hydrochinon-Derivate sind durch die Formel (III) allgemein definiert. In dieser Formel (III) steht R⁴ vorzugsweise für diejenigen Reste, die bereits im Zusammenhang mit der Beschreibung der Verbindungen der Formel (I) vorzugsweise für diesen Substituenten genannt wurden. R steht in Formel (III) vorzugsweise für Wasserstoff, ein Natriumkation, ein Kaliumkation oder ein Äquivalent eines Calciumkations. Besonders bevorzugt steht R für Wasserstoff, ein Natriumkation oder Kaliumkation. Ganz besonders bevorzugt steht R für Wasserstoff.

Die Verbindungen der Formel (III) sind allgemein bekannte Verbindungen der organischen Chemie.

Als Beispiele für die Hydrochinon-Derivate der Formel (III) seien genannt:
Hydrochinon, 2-Methyl-hydrochinon, 2-Ethyl-hydrochinon und die entsprechenden Di-Natrium- bzw. Di-Kaliumsalze.

Das erfindungsgemäße Verfahren zur Herstellung der Verbindungen der Formel (IV) bzw. der erfindungsgemäße Verfahrensschritt (a) wird in Gegenwart von Verdünnungsmitteln durchgeführt. Als Verdünnungsmittel kommen vorzugsweise polare, aprotische organische Lösungsmittel in Frage, wie z.B. Nitrile wie z.B. Acetonitril und Propionitril, Amide wie z.B. Dimethylformamid, Dimethylacetamid, Diethylacetamid und N-Methylpyrrolidon sowie Dimethylsulfoxid, Tetramethylharnstoff, Tetramethylensulfon und Hexamethylphosphorsäuretriamid.

Als Säureakzeptoren für das erfindungsgemäße Verfahren bzw. den erfindungsgemäßen Verfahrensschritt (a) können praktisch alle üblicherweise verwendbaren Säurebindemittel eingesetzt werden. Besonders bewährt haben sich Alkali-und Erdalkalihydroxide, bzw. -oxide wie Natrium- und Kaliumhyroxid, Calciumoxid oder Calcium-hydroxid, Alkali-und Erdalkalicarbonate wie Natrium-, Kalium- und Calciumcarbonat, Alkalialkoholate wie Natrium- oder Kaliummethylat, -ethylat und -tert.-butylat.

Als Mineralsäure kommen für das erfindungsgemäße Verfahren vorzugsweise in Frage: Salzsäure, Essigsäure, Phosphorsäure oder Schwefelsäure.

Die Reaktionstemperatur kann innerhalb eines größeren Bereichs variiert werden. Im allgemeinen arbeitet man zwischen 70°C und 140° C, vorzugsweise bei 90⁰C bis 120°C. Das erfindungsgemäße Verfahren wird im allgemeinen bei Normaldruck durchgeführt.

Zur Durchführung des erfindungsgemäßen Verfahrens zur Herstellung der Verbindungen der Formel (IV) bzw. des Verfahrensschrittes (a) werden auf 1 Mol der Verbindung der Formel (II), 2 bis 2,5 Mol, vorzugsweise 2 bis 2,2 Mol Hydrochinon-Derivat der Formel (III) und gegebenenfalls (für den Fall R = H) 2 bis 2,5 Mol, vorzugsweise 2 bis 2,2 Mol Säureakzeptor einsetzt. Bevorzugt wird die Umsetzung in Gegenwart von Verbindungen der Formel (III), worin R für Wasserstoff steht, und in Gegenwart von Säureakzeptoren durchgeführt. Nach Ablauf der Reaktion wird das Reaktionsgemisch auf Wasser gegossen und mit einer Mineralsäure wie z. B. Salzsäure oder Schwefelsäure angesäuert bis ein pH-Wert = 5 erreicht wird. Die weitere Aufarbeitung geschieht nach allgemein bekannten Methoden.

Verwendet man für das erfindungsgemäße Verfahren bzw. den erfindungsgemäßen Verfahrensschritt (b) 4-(2-Methyl-4-nitro-phenoxy)-phenol als Ausgangsstoff, Tetrachlormethan und Fluorwasserstoff als Reaktionskomponenten, so kann die Reaktion durch das folgende Formelschema wiedergegeben werden:

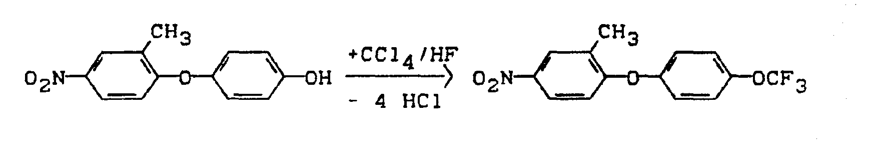

Die beim erfindungsgemäßen Verfahrensschritt (b) als Ausgangsstoffe einzusetzenden Hydrochinon-Derivate sind durch die Formel (IV) allgemein definiert. In dieser Formel stehen R¹, R² und R⁴ vorzugsweise für diejenigen Reste, die bereits im Zusammenhang mit der Beschreibung der Verbindungen der Formel (I) vorzugsweise für diese Substituenten genannt wurden.

Die Verbindungen der Formel (IV) lassen sich gemäß dem erfindungsgemäßen Verfahren bzw. dem erfindungsgemäßen Verfahrensschritt (a) (siehe oben) herstellen. Die Verbindungen der Formel (IV) sind teilweise bekannt (vergl. z. B. CA 102 : 220 227 u). Neu und Bestandteil der vorliegenden Erfindung ist die folgende Verbindung der Formel (IVa)

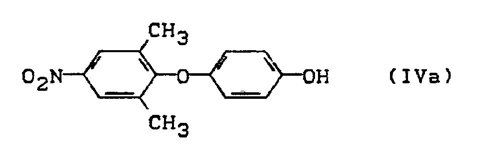

Die Verbindung der Formel (IVa) läßt sich gemäß dem Verfahrensschritt (a) herstellen (siehe Herstellungsbeispiel).

Als Beispiele für die Verbindungen der Formel (IV) seien genannt: 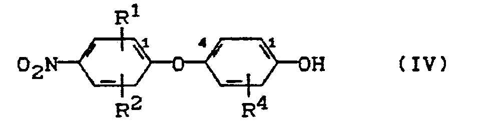
R⁴ = H, 2-CH₃ oder 3-CH₃

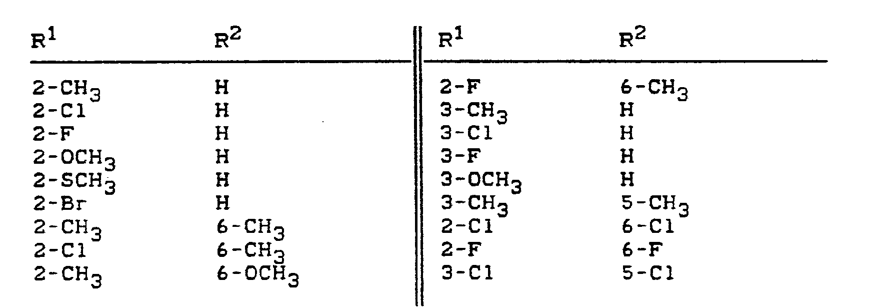

Als halogenierte gesättigte oder ungesättigte Kohlenwasserstoffe kommen in Frage: Tetrachlormethan, Tetrafluorethylen, Dichlordifluorethylen, Chlordifluorethylen, Difluorchlormethan, Dichlorfluormethan und Chlortrifluorethylen.

Als Fluorwasserstoff wird beim erfindungsgemäßen Verfahren (b) im allgemeinen wasserfreie Flußsäure eingesetzt. Der Fluorwasserstoffzusatz ist nur dann erforderlich, wenn in den eingesetzten halogenierten gesättigten Kohlenwasserstoffen ein oder mehrere Halogenatome (vorzugsweise Chloratome) durch Fluor ersetzt werden sollen. In den erfindungsgemäß zu verwendenden ungesättigten Kohlenwasserstoffen, welche an die Verbindungen der Formel (IV) addiert werden, sind die in den Endprodukten befindlichen entsprechenden Fluoratome bereits enthalten, so daß in diesem Fall der Fluorwasserstoffzusatz entfällt.

Das erfindungsgemäße Verfahren (b) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische, gegebenenfalls halogenierte Kohlenwasserstoffe wie Methylenchlorid, Ethylenchlorid, Chloroform, Tetrachlorkohlenstoff, Ether wie Diethyl- und Dibutylether, Glykoldimethylether und Diglykoldimethylether, Tetrahydrofuran und Dioxan, Ketone wie Aceton, Methylethyl-, Methyl-isopropyl- und Methyl-isobutyl-keton, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Bei der Durchführung des Verfahrens (b) in Gegenwart von gesättigten halogenierten Kohlenwasserstoffen wird vorzugsweise in Gegenwart von aprotischen, mit Wasser mischbaren Verdünnungsmitteln gearbeitet wie z. B. Acetonitril, Tetrahydrofuran, Dimethylformamid und Tetramethylensulfon.

Als Basen können bei dem erfindungsgemäßen Verfahren (b) alle üblicherweise für derartige Umsetzungen verwendbaren Basen eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate wie Natrium- und Kaliumcarbonat.

Als Katalysatoren können beim erfindungsgemäßen Verfahren (b) alle üblichen Phasentransferkatalysatoren verwendet werden. Vorzugsweise in Frage kommen quartäre Ammoniumsalze wie z. B. Tetrabutylammoniumchlorid oder -bromid, Triethylbenzylammoniumchlorid und Methyltrioctylammoniumchlorid.

Die Reaktionstemperaturen können bei dem erfindungsgemäßen Verfahren (b) in einem größeren Bereich variiert werden. Im allgemeinen arbeitet man bei Temperaturen zwischen 0 °C und 160 °C, vorzugsweise bei Temperaturen zwischen 20 °C und 140 °C.

Das erfindungsgemäße Verfahren (b) wird im allgemeinen im Druckbereich von Normaldruck bis 40 bar, bevorzugt von Normaldruck bis 15 bar, durchgeführt. Die erfindungsgemäßen Drucke stellen sich beispielsweise durch den Eigendruck ein, der entsteht, wenn man im erfindungsgemäßen Temperaturbereich das Verfahren in einem Autoklaven durchführt.

Bei der Durchführung des erfindungsgemäßen Verfahrens (_{b}) werden die Flußsäure und der halogenierte, gesättigte Kohlenwasserstoff im großen Überschuß zugegeben. Vorzugsweise setzt man auf 1 Mol der Verbindungen der Formel (IV) 5 bis 100Mol, vorzugsweise 10 bis 75 Mol, Flußsäure und gesättigten Kohlenwasserstoff ein.

Auch die ungesättigten halogenierten Kohlenwasserstoffe werden im allgemeinen in einem großen Überschuß eingesetzt. Vorzugsweise wird so viel ungesättigter halogenierter Kohlenwaserstoff gasförmig in das Reaktionsgemisch eingeleitet bis keine weitere Aufnahme mehr erfolgt.

Das erfindungsgemäße Verfahren (b) kann beispielsweise wie folgt durchgeführt werden:

In einem Autoklaven legt man die Verbindung der Formel (IV), den halogenierten Kohlenwasserstoff und die Flußsäure vor. Das Reaktionsgemisch wird gegebenenfalls in einer Stickstoffatmosphäre auf die Reaktionstemperatur erhitzt, wobei sich der entsprechende Reaktionsdruck einstellt.

Nach Beendigung der Umsetzung wird der Restdruck entspannt und das Reaktionsgemisch nach allgemeinen Methoden aufgearbeitet.

Die Herstellung der Verbindungen der Formel (Ib) bzw. ihre Herstellung gemäß Verfahrensschritt (c) ist ebenfalls Teil der vorliegenden Erfindung.

Es wurde demgemäß gefunden, daß man die Verbindungen der Formel (Ib) 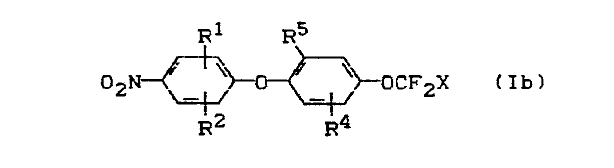 in welcher
X, R¹, R² und R⁴ die oben angegebenen Bedeutungen haben und
_{R}⁵ für Chlor oder Brom steht,
erhält,
wenn man die Verbindungen der Formel (la) 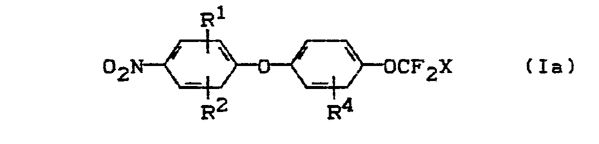 in welcher
X, R¹, R² und R⁴ die oben angegebenen Bedeutungen haben,

in Gegenwart von Katalysatoren und in Gegenwart von polaren Verdünnungsmitteln bei Temperaturen zwischen 0 °C und 100 °C halogeniert und die Verbindungen der Formel (Ib) isoliert.

Verwendet man für das erfindungsgemäße Verfahren bzw. den erfindungsgemäBen Verfahrensschritt (c) 4-(2-Methyl-4-nitro-phenoxy)-trifluormethoxybenzol als Ausgangsstoff und chloriert in Gegenwart von Eisen-(III)-chlorid als Katal^{y-} sator, so kann die Reaktion durch das folgende Formelschema wiedergegeben werden:

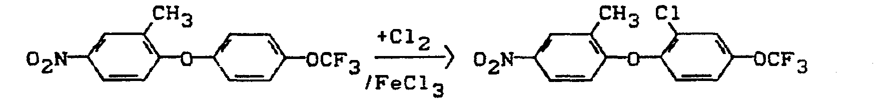

Überraschenderweise kann man mit Hilfe des erfindungsgemäßen Verfahrensschrittes (c) eine selektive Monohalogenierung durchführen, wobei die Reaktionsprodukte in sehr guten Ausbeuten und in hoher Reinheit erhalten werden. Nach allgemeinem Stand der Technik wäre eine selektive Monohalogenierung unter den Reaktionsbedingungen nicht zu erwarten gewesen.

Die beim erfindungsgemäBen Verfahrensschritt (c) als Ausgangsstoffe einzusetzenden Verbindungen sind durch die Formel (Ia) allgemein definiert. In dieser Formel haben _{X}, _{R}^{l}, _{R}² und R⁴ vorzugsweise diejenigen Bedeutungen, die bereits im Zusammenhang mit der Beschreibung der Verbindungen der Formel (I) vorzugsweise für diese Substituenten genannt wurden.

Die Verbindungen der Formel (Ia) lassen sich gemäß dem erfindungsgemäßen Verfahren bzw. Verfahrensschritt (b) und/oder nach allgemein bekannten Methoden herstellen (vergl. z. B. US-PS 4 192 669).

Als Halogenierungsmittel wird für das erfindungsgemäße Verfahren Chlor oder Brom eingesetzt. Vorzugsweise wird in Gegenwart von Chlor halogeniert.

Das erfindungsgemäße Verfahren (c) zur Herstellung der Verbindungen der Formel (Ib) wird vorzugsweise unter Verwendung von Verdünnungsmitteln durchgeführt. Als Verdünnungsmittel kommen dabei praktisch alle organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole wie Methanol, Ethanol, n- oder i-Propanol und niedere Carbonsäuren wie Essigsäure oder Propionsäure. Bevorzugt wird ein Gemisch aus Essigsäure und Methanol eingesetzt.

Als Katalysatoren werden beim erfindungsgemäßen Verfahren (c) alle üblichen Halogenübertrager eingesetzt. Vorzugsweise werden Lewis-Säuren wie z. B. Eisen-(III)-chlorid, metallisches Eisen oder Aluminiumchlorid verwendet. Besonders bevorzugt wird Eisen-(III)-chlorid eingesetzt.

Die Reaktionstemperaturen können bei dem erfindungsgemäßen Verfahren (c) in einem größeren Bereich variiert werden. Im allgemeinen arbeitet man bei Temperaturen zwischen 0 °C und 100 °C, vorzugsweise bei Temperaturen zwischen 20 °C und 80 °C .

Das erfindungsgemäße Verfahren (c) wird im allgemeinen unter Normaldruck durchgeführt. Es ist jedoch auch möglich, unter erhöhtem oder vermindertem Druck zu arbeiten.

Bei der Durchführung des erfindungsgemäßen Verfahrens (c) setzt man auf 1 Mol der Verbindung der Formel (Ia) 0,01 Mol bis 1 Mol, vorzugsweise 0,01 Mol bis 0,6 Mol Katalysator ein. Bei der Durchführung des Verfahrens (c) geht man im allgemeinen so vor, daß eine Lösung von der Verbindung der Formel (Ia) in einem Verdünnungsmittel mit einem Katalysator versetzt wird und bis zur Sättigung gasförmiges Halogen eingeleitet wird. Die Aufarbeitung der entstehenden Reaktionsprodukte geschieht nach allgemein üblichen Methoden.

Die Verbindungen der Formel (Ib) die nach dem oben beschriebenen Verfahren (c) erhalten werden, sind zum Teil neu. Neu und Teil der vorliegenden Erfindung sind die folgenden Verbindungen der Formel (Ic): 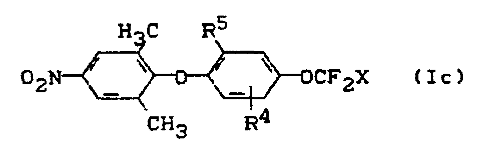 in welcher
R⁴ und X die für die Formel (1) angegebenen Bedeutungen haben und
R⁵ für Chlor oder Brom steht.

Die Verbindungen der Formel (I) die nach den oben beschriebenen Verfahrensschritten (a) und (b) bzw. (a), (b) und (c) hergestellt werden können, sind wichtige Zwischenprodukte für die Herstellung von Pestiziden, wie Insektiziden und Herbiziden. So lassen sich Verbindungen der Formel (I) z. B. zu den entsprechenden Anilinen reduzieren, die man anschließend mit Isocyanaten zu den entsprechenden Harnstoffen umsetzt (vergl. z. B. EP-A-57 888, EP-A-93 976, EP-A-93 977, EP-A-132 680 und EP-A-122 449 entsprechend US-Anmeldung Ser. Nr. 587 088) oder für die Herstellung von bekannten gut wirksamen Herbiziden wie z.B. Phenoxypropionsäure-Derivaten verwenden (vergl. z.B. US-PS 4 192 669).

### Herstellungsbeispiele

### Beispiel 1

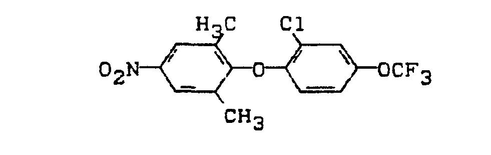

20 g (0,061 Mol) 4-(2,6-Dimethyl-4-nitro-phenoxy)-tri- fluormethoxybenzol werden in 100 ml Eisessig und 25 ml Methanol gelöst und mit 3,5 g (0,03 Mol) Eisen-(III)-chlorid versetzt. Anschließend wird bis zur Sättigung und bei einer Temperatur von 40 °C bis 50 °C Chlorgas eingeleitet und ca. 16 Stunden bei dieser Temperatur gerührt. Das Lösungsmittel wird abdestilliert, in Methylenchlorid aufgenommen und einmal mit Hydrogencarbonatlösung gewaschen. Anschließend wird die organische Phase getrocknet, eingeengt und andestilliert.

Man erhält 21,2 g (96 % der Theorie) 3-Chlor-4-(2,6-dimethyl-4-nitro-phenoxy)-trifluormethoxybenzol als kristalliner Rückstand mit einer Reinheit von 91 % und einem Schmelzpunkt von 54 °C - 56 °C.

### Beispiel 2

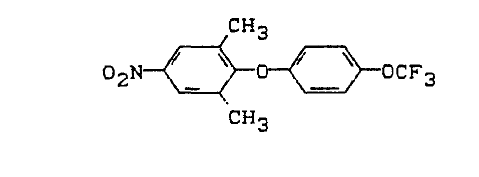

In einer Fluorierungsapparatur werden bei 0°C - 10 °C 200 ml Flußsäure, 45 g 95 prozentiges 4-(2,6-Dimethyl-4-nitro-phenoxy)-phenol und 250 ml Tetrachlormethan vorgelegt, ca. 3 bar Stickstoff aufgedrückt und 6 Stunden bei 120 °C erhitzt, wobei der entstehende Chlorwasserstoff kontinuierlich entspannt wird. Nach Abkühlen und Abdestillieren der nicht verbrauchten Flußsäure wird der Rückstand mit Wasser gewaschen, getrocknet und destilliert. Die Fraktion bei 140 °C - 155 °C/0,4 mbar enthält zu 81 X das gewünschte Produkt.

Man erhält nach Kristallisation aus n-Hexan 28 g (52 % der Theorie) 4-(2,6-Dimethyl-4-nitro-phenoxy)-trifluormethoxy- benzol mit einer Reinheit von 92 % und einem Schmelzpunkt von 66 °C - 67 °C.

### Beispiel 3

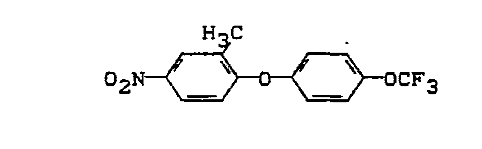

In einer Fluorierungsapparatur werden bei 0 °C - 10 °C 400 ml Flußsäure, 100 g (0,4 Mol) 4-(2-Methyl-4-nitro- phenoxy)-phenol und 500 ml Tetrachlormethan vorgelegt, ca. 3 bar Stickstoff aufgedrückt und 7 Stunden bei 120 °C und 28 bar Eigendruck zur Reaktion gebracht.

Man erhält nach destillativer Aufarbeitung 67 g (52,5 % der Theorie) 4-(2-Methyl-4-nitro-phenoxy)-trifluormethoxy- benzol vom Siedepunkt Kp: 138 °C - 142 °C/0,2 mbar.

### Bei^{p}iel 4

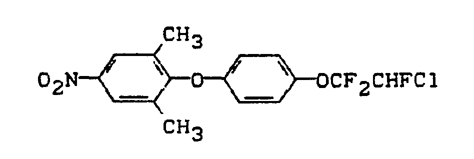

In 200 ml Dimethylformamid werden 10 g Kaliumhydroxid und 40 g (0,15 Mol) 4-(2,6-Dimethyl-4-nitro-phenoxy)-phenol bei 55°C - 60 °C vorgelegt und Trifluorchlorethylen eingeleitet bis nichts mehr aufgenommen wird. Nach Abkühlen werden 500 ml Wasser eingerührt und mit Toluol extrahiert. Die organische Phase wird mit Wasser gewaschen, eingeengt, getrocknet und das Rohprodukt wird anschließend destilliert.

Man erhält 35 g (62 X der Theorie) 4-(2,6-Dimethyl-4-nitro-phenoxy)-(2-chlor-1,1,2-trifluorethoxy)-benzol vom Siedepunkt Kp: 180 °C - 190 °C/1,1 mbar.

### Beispiel 5

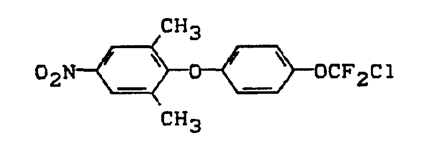

Analog Beispiel 2 erhält man aus 50 g (0,19 Mol) 4-(2,6-Dimethyl-4-nitro-phenoxy)-phenol in 100 ml Dichlormethan, 200 ml Flußsäure und 200 ml Tetrachlormethan, 34 g (51 % der Theorie) 4-(2,6-Dimethyl-4-nitro-phenoxy)-chlordi- fluormethoxybenzol vom Siedepunkt Kp: 160 °C - 165 °C/ 0,3 mbar.

### Beispiel 6

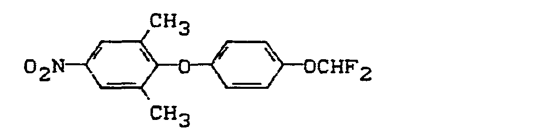

In 100 ml Dioxan werden 26 g (0,1 Mol) 4-(2,6-Dimethyl-4-nitro-phenoxy)-phenol vorgelegt und 25 g 50 prozentige Natronlauge zugegeben. Bei einer Temperatur von 55 °C - 60 °C wird bis zur Sättigung Chlordifluormethan eingeleitet. Anschließend wird Dioxan im Vakuum abdestilliert, der Rückstand in 100 ml Dichlormethan und 150 ml Wasser gelöst, alkalisch gestellt und mit Toluol extrahiert. Nach dem Trocknen wird eingeengt und über eine Kieselgelsäule chromatographiert.

Man erhält 16 g (52 % der Theorie) 4-(2,6-Dimethyl-4-nitro-phenoxy)-difluormethoxybenzol (Idendifikation durch ¹H-, ¹⁹F- und Massen-Spektren).

### Herstellung von Verbindungen der Formel (IV)

### Beispiel (IV-1)

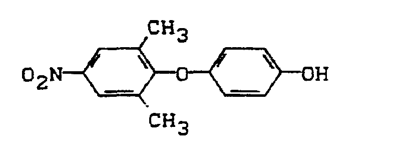

365 g (3,32 Mol) Hydrochinon und 187,5 g (3,34 Mol) Kaliumhydroxid werden in 1660 ml Dimethylsulfoxid gelöst und bei ca. 90 °C mit 308 g (₁,66 Mol) 2,6-Dimethyl-4-nitro-chlorbenzol versetzt. Man rührt 4 Stunden bei 100 °C nach und gießt nach Abkühlen anschließend die Reaktionsmischung in 20 1 Wasser. Unter Rühren versetzt man mit konzentrierter Salzsäure bis ein pH-Wert von 5 erreicht ist. Das ausgefallene Produkt wird abgesaugt und getrocknet.

Man erhält so 373,4 g (87,3 % der Theorie) 4-(2,6-Dimethyl-4-nitro-phenoxy)-phenol mit einem Gehalt von 97,5 %.

## Patentansprüche

1. Verfahren zur Herstellung von Halogenalkoxydiphenylether der Formel (I) 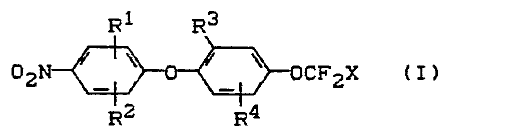 in welcher
R¹ für Wasserstoff, Halogen, Alkyl, Alkoxy oder Alkylthio steht,
R² für Wasserstoff, Halogen, Alkyl, Alkoxy oder Alkylthio steht,
R³ für Wasserstoff, Chlor oder Brom steht,
R⁴ für Wasserstoff oder Alkyl steht und
X für Wasserstoff, Fluor, Chlor oder die Reste -CH₂Cl, -CHF₂, -CHCl₂ und -CHFC1 steht,
dadurch gekennzeichnet, daß man
(a) Halogenbenzole der allgemeinen Formel (II) 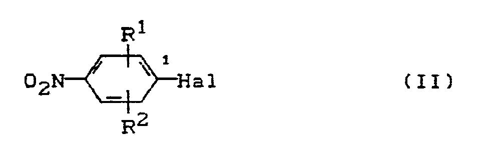 in welcher
R¹ und R² die oben angegebenen Bedeutungen haben und
Hal für Halogen steht,
mit Hydrochinon-Derivaten der allgemeinen Formel (III) 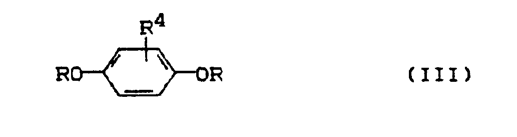 in welcher
R⁴ die oben angegebenen Bedeutungen hat und
R für Wasserstoff oder ein Äquivalent eines Alkali- oder Erdalkalimetallkations steht,
gegebenenfalls in Gegenwart von Säureakzeptoren und in Gegenwart von inerten Verdünnungsmitteln bei Temperaturen zwischen 70 °C und 140 °C umsetzt, gegebenenfalls mit Mineralsäuren versetzt zu den Hydrochinonmonophenylethern der allgemeinen Formel (IV) 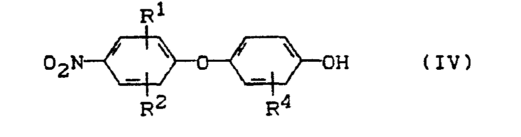 in welcher
_{R}¹, R² und R⁴ die oben angegebenen Bedeutungen haben,
und anschließend
(b) die Verbindungen der Formel (IV), nach ihrer Isolierung, mit halogenierten gesättigten oder ungesättigten Kohlenwasserstoffen, gegebenenfalls in Gegenwart von Fluorwasserstoff, gegebenenfalls in Gegenwart von Basen, gegebenenfalls in Gegenwart von Katalysatoren und gegebenenfalls in Gegenwart von Verdünnungsmitteln bei Temperaturen zwischen 0 °C und 160 °C zu den Verbindungen der Formel (Ia) 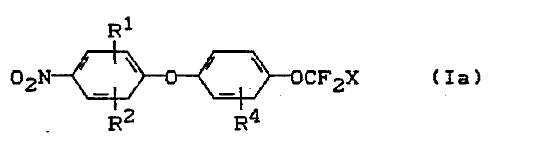 in welcher
X, R¹, R2 und R⁴ die oben angegebenen Bedeutungen haben,
umsetzt und gegebenenfalls anschließend
(c) die Verbindungen der Formel (Ia), nach ihrer Isolierung, in Gegenwart von Katalysatoren und in Gegenwart von polaren Verdünnungsmitteln bei Temperaturen zwischen 0 °C und 100 °C halogeniert und die Verbindungen der Formel (Ib) 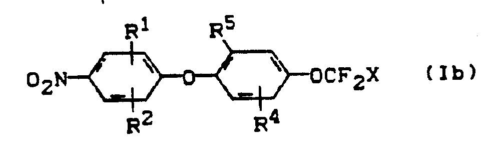 in welcher
X, R¹, R² und R⁴ die oben angegebenen Bedeutungen haben und
R^{S} für Chlor oder Brom steht,
isoliert.

2. Verfahren zur Herstellung von Hydrochinonmonophenylethern der allgemeinen Formel (IV) 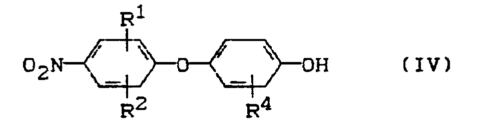 in welcher
_{R}¹, _{R}² und R⁴ die in Anspruch 1 angegebenen Bedeutungen haben,
dadurch gekennzeichnet, daß man Halogenbenzole der allgemeinen Formel (II) 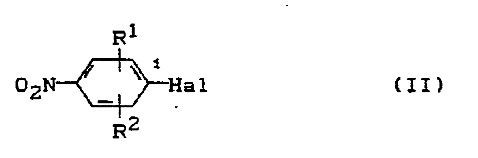 in welcher
R¹, R² und Hal die in Anspruch 1 angegebenen Bedeutungen haben,
mit Hydrochinon-Derivaten der allgemeinen Formel (III) 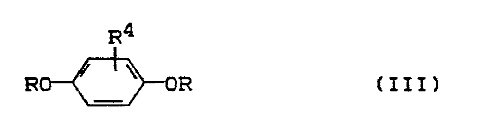 in welcher
R und R⁴ die in Anspruch 1 angegebenen Bedeutungen haben,
gegebenenfalls in Gegenwart von Säureakzeptoren und in Gegenwart von inerten Verdünnungsmitteln bei Temperaturen zwischen 70°C und 140°C umsetzt, anschließend gegebenenfalls mit Mineralsäuren versetzt und die Verbindungen der Formel IV isoliert.

3. Verfahren zur Herstellung der Verbindungen der Formel (Ib) 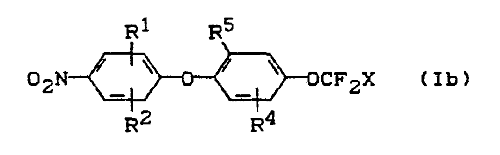 in welcher
X, R¹,-R² und R⁴ die in Anspruch 1 angegebenen Bedeutungen haben und
R⁵ für Chlor oder Brom steht,
dadurch gekennzeichnet, daß man die Verbindungen der Formel (Ia) 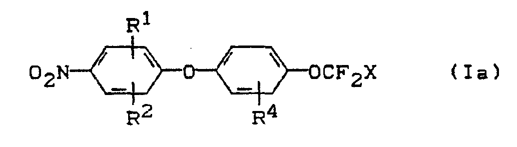 in welcher
X, R¹, R² und R⁴ die die in Anspruch 1 angegebenen Bedeutungen haben,
in Gegenwart von Katalysatoren und in Gegenwart von polaren Verdünnungsmitteln bei Temperaturen zwischen 0 °C und 100 °C halogeniert und die Verbindungen der Formel (Ib) isoliert.

4. Verfahren gemäß den Ansprüchen 1 bis 3, wobei in den allgemeinen Formeln
R¹ für Wasserstoff, Halogen wie Fluor, Chlor oder Brom und für Alkyl, Alkoxy oder Alkylthio mit jeweils 1 bis 6 Kohlenstoffatomen steht,
R² für Wasserstoff, Halogen wie Fluor, Chlor oder Brom und für Alkyl, Alkoxy oder Alkylthio mit jeweils 1 bis 6 Kohlenstoffatomen steht,
R³ für Wasserstoff, Chlor oder Brom steht,
R⁴ für Wasserstoff oder Alkyl mit 1 bis 6 Kohlenstoffatomen steht und
X für Wasserstoff, Fluor, Chlor oder die Reste -CH₂Cl, -CHF₂, -CHCl₂ und -CHFC1 steht.

5. Verfahren gemäß den Ansprüchen 1 bis 3, wobei in den allgemeinen Formeln
R¹ für Wasserstoff, Fluor, Chlor, Brom oder für Alkyl, Alkoxy oder Alkylthio mit jeweils 1 bis 4 Kohlenstoffatomen steht,
R² für Wasserstoff, Fluor, Chlor, Brom oder für Alkyl, Alkoxy oder Alkylthio mit jeweils 1 bis 4 Kohlenstoffatomen steht,
R³ für Wasserstoff, Chlor oder Brom steht,
R⁴ für Wasserstoff, oder Alkyl mit 1 bis 4 Kohlenstoffatomen steht und
X für Wasserstoff, Fluor, Chlor oder die Reste -CH₂Cl, -CHF₂, -CHC1₂ und -CHFC1 steht.

6. Verfahren gemäß den Ansprüchen 1 bis 3, wobei in den allgemeinen Formeln
R¹ für Wasserstoff, Fluor, Chlor, Methyl, Methoxy und Methylthio steht,
R² für Wasserstoff, Fluor, Chlor, Methyl, Methoxy und Methylthio steht,
R³ für Wasserstoff oder Chlor steht,
R⁴ für Wasserstoff, Methyl oder Ethyl steht und

X für Wasserstoff, Fluor, Chlor oder die Reste -CH₂Cl, -CHF₂, -CHC1₂ und -CHFC1 steht.

7. Verfahren gemäß den Ansprüchen 1 bis 3, wobei in den allgemeinen Formeln
R¹ für Methyl in der 2-Stellung des Nitrophenylrestes steht,
R² für Wasserstoff oder Methyl in der 6-Stellung des Nitrophenylrestes steht,
R³ für Wasserstoff oder Chlor steht,
R⁴ für Wasserstoff steht, und
X für Wasserstoff, Fluor, Chlor oder den Rest CHFC1 steht.

8. Verbindung der Formel

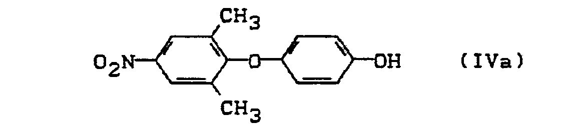

9. Verbindungen der Formel 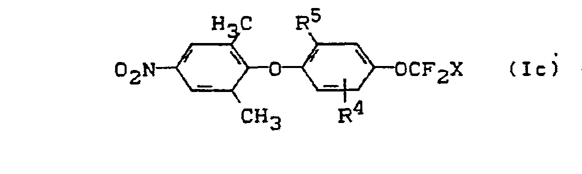 in welcher
R⁴ und X die für die Formel (I) in Anspruch 1 angegebenen Bedeutungen haben und
R⁵ für Chlor oder Brom steht.

10. Verbindungen gemäß Anspruch 9, wobei
R⁴ für Wasserstoff steht und
X für Wasserstoff, Fluor, Chlor oder CHFC1 steht.
